# EUROPEAN PATENT SPECIFICATION

(11) **EP 1 046 713 B1**
(45) Date of publication and mention of the grant of the patent: **03.09.2008**
(21) Application number: 00103484.2
(22) Date of filing: 01.03.2000
(51) Int. Cl.: A61K 31/00, A61K 39/07, A61K 39/09, C12N 1/00, C12N 1/20, C12Q 1/04, C12Q 1/16

(54) **Method for the selection of adhesive lactobacilli strains having therapeutical properties and strains obtained by said method**
Verfahren zur Auswahl von klebenden Lactobacillus-Stämmen mit therapeutischen Eigenschaften und durch diese Methode erreichte Stämme
Méthode pour la sélection des souches adhérentes de Lactobacillus ayant des propriétés thérapeutiques et souches obtenues par cette méthode

(30) Priority: 23.04.1999 IT MI990858
(43) Date of publication of application: 25.10.2000
(73) Proprietor: Proge Farm S.r.l., 28065 Cerano (NO) (IT)
(72) Inventor: Dondi, Giancarla, 28065 Cerano (NO) (IT)
(74) Representative: Gislon, Gabriele

(56) References cited:
- EP-A- 0 861 905
- US-A- 5 032 399
- BORIS S ET AL: "Adherence of human vaginal lactobacilli to vaginal epithelial cells and interaction with uropathogens" INFECTION AND IMMUNITY, AMERICAN SOCIETY FOR MICROBIOLOGY. WASHINGTON, US, vol. 66, no. 5, May 1998 (1998-05), pages 1985-1989, XP002958494 ISSN: 0019-9567
- REID G ET AL: "Influence of lactobacilli on the adhesion of Staphylococcus aureus and Candida albicans to fibers and epithelial cells" JOURNAL OF INDUSTRIAL MICROBIOLOGY, vol. 15, no. 3, 1995, pages 248-253, XP009023114 ISSN: 0169-4146
- REID GREGOR ET AL: "Use of lactobacilli to reduce the adhesion of Staphylococcus aureus to catheters" INTERNATIONAL BIODETERIORATION AND BIODEGRADATION, vol. 34, no. 1, 1994, pages 73-83, XP000971024
- KOTARSKI S F ET AL: "MODELS FOR STUDY OF THE SPECIFICITY BY WHICH INDIGENOUS LACTOBACILLI ADHERE TO MURINE GASTRIC EPITHELIA" INFECTION AND IMMUNITY, vol. 26, no. 3, 1979, pages 966-975, XP009023112 ISSN: 0019-9567
- DATABASE BIOSIS [Online] BIOSCIENCES INFORMATION SERVICE, PHILADELPHIA, PA, US; 1997 KALANTZOPOULOS G: "Fermented products with probiotic qualities" Database accession no. PREV199799754915 XP002264967 & ANAEROBE, vol. 3, no. 2-3, 1997, pages 185-190, ISSN: 1075-9964

## Description

The present invention relates to new strains of *Lactobacillus plantarum, Lactobacillus acidophilus, Lactobacillus delbrueckii, Streptococcus thermophilus*, their use and pharmaceutical compositions containing them.

### TECHNOLOGICAL BACKGROUND

Lactic bacteria are widely distributed in nature. A number of species are present in alimentary products (milk, yogurt, fruits, vegetables and many others), whilst only some strains are usual eubiotic saprophytes of the intestinal and/or urogenital flora.

Generally known lactic bacteria, when administered by oral or vaginal or by other routes, restore the physiological bacterial flora.

In order to exert their activity over a long time, lactobacteria must be able to adhere to mucosal epithelial cells with which they come into contact in such way to form a biofilm that, fixed on epithelial cells, constitutes a real barrier between the epithelium and possible pathogen bacteria.

Said evidence shows that the adhesion ability of lactic bacteria, recovered from various human habitats, is an essential requirement for them to exert their prophylactic beneficial activity as, besides improving the capability to exert their action, they can avoid toxygen infections by preventing the adhesion of pathogens to the tissue surface.

It is commonly acknowledged that bacterial adhesion to the epithelial mucosa represents an important requirement for bacterial colonization of specific sites in plants and animals, namely by exerting a main role in the human normal bacterial flora physiology and in the pathogenesis of human infections caused by pathogenic bacteria.

The specificity of the interaction between bacteria and host tissue is confirmed by the species-specificity of the localization, in particular sites of the host, of the normal saprophytic flora and of some bacterial infections.

The ability of microorganisms to adhere to epithelial cells is moreover also host-specific: in fact it has been verified that particular strains of lactic bacteria are specific colonizers of some species of animals and not of other ones.

Boris S et al, Infection and Immunity, American Society for Microbiology, Washington, US, vol 66, no. 5, May 1998, pages 1985-1989 discloses competition tests between lactobacilli and well known pathogens.

Reid G et al. Journal of Industrial Microbiology, vol. 15, no. 3, 1995, pages 248-253, and Raid Gregor et al., International Biodeterioration and Biodegradation, vol. 34, no. 1, 1994 pages 73-83, report on the effect of coincubation of lactobacilli and pathogens.

Kotarski SF et al., Infection and Immunity, vol. 26, no. 3, 1979, pages 966-975, discloses the liquid scintillation technique to count the number of lactobacilli adhered in vitro to murine gastric cells.

Kalantzopoulos G ,1997, Biosis Database Acc No. XP002264967 discloses some of the bacterial species used as priobiotics, i.a. Streptococcus, Lactobacillus and Bifidobaterium species.

US 5.032.399 discloses for example a particular strain of *Lactobacillus acidophilus* isolated from the normal intestinal flora, quoted as *Lactobacillus GG,* and subsequently reclassified as *Lactobacillus casei subsp. Rhamnosus,* of which it reports, besides other characteristics, the capability of said strain to adhere to human intestinal mucosa cells.

In the above mentioned patent a method suitable to assess "in vitro" whether the tested strain possesses the ability to adhere to isolated intestinal mucosa cells is disclosed.

More in general, the evaluation of the ability to colonize and/or to adhere to epithelia is carried out either by in vitro systems (adhesion to cell lines and to organ cultures) or by the administration of the strains to be examined to healthy volunteers. In literature, studies have been reported showing further confirmation of the fact that the different adhesion ability is typical of a single strain and not of species, as it is deemed to be important from a general point of view, in the adhesion mechanism, the nature of the surface on which the microorganism must adhere, but above all, the physiological, biochemical and genetic characteristics of the microorganism concerned.

In that way it can be predicted how to select in the population taken into account strains which mostly show to possess said adhesion ability. The mechanism and the factors that regulate adhesion ability are not yet completely made clear. Some authors state that in the adhesion mechanism chemical bonds between surfaces are important; other authors disclose that carbohydrate layers, covering bacterial cells, mediate the fixation; other ones foresee the presence of acidic mucopolysaccharides or complex macromolecules containing teicoic acid or exocellular polymers; other ones further disclose, as key-factor for the adhesion, the presence of surface bacterial antigens known as adesines, often constituted by filamentous projections made by pili or fimbriae departing from the carbohydrate layer wrapped around the bacterial cells.

In conclusion, microorganisms adhering to the epithelial surface represent an important element for the health and the well-being as they constitute a bar between the epithelium and patogens.

For this reason the selection of lactic bacteria strains having adhesive ability allows selected strains to exert prophylactic or restoring activity.

Some lactic bacteria strains isolated from intestinal and/or urogenital habitat, after selection on a laboratory scale, although showing in vitro adhesive properties, are not able to adhere in vivo to physiological epithelia. This is due to the possibility of the incidental presence of already installed pathogens (or not yet installed but with high adhesion capacity for the type of epithelium considered), preventing the adhesion and the subsequent colonization of lactic bacteria.

Therefore, to be sure to deal with lactic bacteria that can exert their therapeutic activity, strains must be previously selected in order to identify those that can guarantee in vivo the ability to compete with pathogens.

### DISCLOSURE OF THE INVENTION

It has now been found a process allowing to select lactic bacteria strains, useful in the treatment of disorders of gastrointestinal or urogenital tracts in man and woman. The method includes an adhesion test of previously isolated and characterized lactic bacteria strains, possibly also freeze-dried and rehydrated in mixture with pathogenic strains, (in a rate from 1:10 to 10:1) and the subsequent selection of the sole bacterial colonies showing strong adhesion ability by competing with the pathogen.

Lactobacilli strain to be submitted to this treatment come from the normal bacterial flora, preferably intestinal and /or uro-vaginal, of a healthy human subject, and they are typically obtained from biological samples containing several bacterial species, isolated by conventional techniques.

Before being submitted to the method , such samples are generally submitted to suitable previous treatments, according to conventional methods, that allow the preservation of the strains up to the moment of their use.

Lactobacteria, optionally previously submitted to conventional adhesion tests, as for instance the one disclosed in US patent 5.032.399, are mixed with pathogen strains incubating them along with isolated intestinal cells.

Lactic bacteria and pathogenic strains adhered to intestinal cells are then resuspended, filtered and washed. It is proceeded afterwards to the count and to the identification of the adhered lactic bacteria.

Bacterial strains to be submitted to the present process, preferably coming from the normal saprophytic flora of healthy subjects, are incubated with a nutrient medium and strains selected after incubation are characterized and kept in a suitable case to be then optionally submitted to the selection methods disclosed in EP A 0861905.

An adhesion test is then carried out by isolating at first suitable human cells, for instance gastrointestinal or vaginal cells, according to conventional methods.

By way of example, an analysis method to evaluate bacterial adhesion in vitro is herein disclosed. Such method involves the collection of effluent from a patient with a well-functioning ileostomy by saline lavage.

The saline effluent is collected in cold 1/10 buffer I (0,1% gelatin, 1% glucose, 500 mM sodium phosphate, pH 7,4). This solution is then filtered through a sieve having diameter 25 to remove clumps and debris afterwards the small intestinal (ileal) mucosa cells collected by centrifuging at 100 x g for 10 min. The cells are then washed with buffer II (0,45% NaCl, 0,1% glucose, 0,01% gelatin, 50 mM sodium phosphate, pH 7,4) using the same centrifuging conditions. Cells are then resuspended in buffer II, some tissue culture medium is added, and the cells are finally stored at 4°C up to the moment of their use.

The number and the viability of ileal cells are determined by Trypan Blue staining. The ileal human cells can be frozen in liquid nitrogen after addition of 10% (v/v) glycerol.

Lactic bacteria are grown for 18 hours at the relative optimal temperatures (comprised between +37 and +42 °C) in MRS or M17 broth or other suitable medium, with addition of ³H-alanine and ³H-leucine. Lactic bacteria are then washed in 5 volumes of phosphate buffered saline, resuspended in buffer II and filtered through 2.0 µ polycarbonate filters to remove clumps. Lactic bacteria are mixed with the pathogens in rate of 1:1, the mixture is then mixed with the isolated intestinal cells and incubated at suitable temperatures (37-42°C), for a period comprised between 1 and 10 minutes, preferably for about 5 minutes. Samples are placed on 2.0 µ polycarbonate filter and the exceeding liquid is removed by weak suction. Filters are then rinsed, first with 2 ml then with 5 ml of phosphate buffered saline (pH 7,4). Using these filters ileal cells and lactic bacteria and the pathogens adhered thereon are retained while free bacteria pass through. Controls include the reaction mixture without human ileal cells. The retained radioactivity is evaluated by liquid scintillation.

Lactic bacteria specific activity is determined from the colony forming units calculated from the optical density at 550 nm and from the radioactivity retained by the 2.0 µ filters . The bacterial specific activity is used to calculate the number of bacteria found. The strains that are bound at a rate of 50, and more preferably 100, bacteria per gastrointestinal cell are deemed to satisfy the criterion of selection.

The present process allows to select lactic bacteria, such as the strains deposited at the LMG of Gent as quoted hereinafter, endowed with high ability of adhesion to human physiological epithelia; particularly strains thus selected have the advantageous property to be able to stick to mucosal tissues competing with the pathogens.

Moreover strains obtained with the process are unexpectedly endowed with the ability of displacing, in vivo, pathogens already installed on the physiological tissues.

Samples of bacteria strains selected by the method above have been deposited according to the Budapest Treaty at the Belgian Coordinated Collection of Microorganisms - BCCM LMG Collection under the following accession numbers:
*Lb. plantarum,* isolated from human vagina: LMG-P-17630 of 29.11.1996
*Lb. acidophilus,* isolated from human gut: LMG-P-18806 of 15.01.1999
*Lb. delbreuckii,* isolated from human gut: LMG-P-18805 of 15.01.1999
*Str. thermophilus*, LMG-P-18807 of 15.01.1999

Such strains constitute a subject-matter of the invention, thanks to their high adhesion ability, in competition with pathogenic bacteria, to human epithelia.

The taxonomic characterization was carried out using conventional methods such as carbohydrate fermentation tests of (API 50 CHL^{®}, Biomerieux), analysis of cell morphology, Gram, oxidase and catalase reactions, SDS-PAGE analysis and "Cluster analysis".

Herein below some characteristics of the strains of the invention are reported.

| *Lactobacillus plantarum* P-17630 | |
|---|---|
| - Cell morphology | single, rarely double, immobile, asporigenic rods |
| - Gram staining | positive |
| - Oxidase reaction | negative |
| - Catalase reaction | negative |
| - Fermentation of sugars positive for: | L-Arabinose, Ribose, Galactose, D-Glucose, D-Fructose, D-Mannose, Ramnose (+/-), Dulcitol, Mannitol, Sorbitol, N-acetil-glucosamine (+/-), Amigdalin (+/-), Arbutin (+/-), Esculin, Salicin (+/-), Cellobiose, Maltose, Lactose, Melibiose, Saccharose, Threalose, Melizitose, D-Raffinose, β-Gentibiose (+/-), D-Turanose, D-Tagatose. Gluconate (+/-). |

| *Lactobacillus acidophilus* P-18806 | |
|---|---|
| - Cell morphology | single, double, immobile, asporigenic rods |
| - Gram staining | positive |
| - Oxidase reaction | negative |
| - Catalase reaction | negative |
| - Fermentation of sugars positive for: | Galactose (+/-), D-Glucose, D-Fructose, D-Mannose, Cellobiose (+/-), Maltose, Lactose (+/-), Saccharose, Trealose (+/-), β-Gentibiose (+/-). |

| *Lactobacillus delbrueckii* P-18805 | |
|---|---|
| - Cell morphology | single, double, immobile, asporigenic rods |
| - Gram staining | positive |
| - Oxidase reaction | negative |
| - Catalase reaction | negative |
| - Fermentation of sugars positive for: | D-Glucose, D-Mannose, ' N-acetil-glucosamine, Lactose, Saccharose (+/-), Threalosio. |

| *Streptococcus thermophilus* P-18807 | |
|---|---|
| - Cell morphology | single, double, chained, immobile, asporigenic cocci |
| - Gram staining | positive |
| - Oxidase reaction | negative |
| - Catalase reaction | negative |
| - Fermentation of sugars | |
| positive for: | D-Glucose, D-Fructose (+/-), D-Mannose (+/-), Lactose, Saccharose. |

The results obtained in vitro can be reproduced in vivo administering the cultures of said lactobacilli, freeze-dried according to known techniques, obtained according to the method disclosed above.

Lactobacilli according to the present invention, con be administered as such or added to a pharmaceutically or anyway physiologically acceptable carrier, thus obtaining various kind of preparations or formulations, such as pharmaceutical compositions, which are further objects of the present invention.

Thus for instance, the claimed bacterial strains can be administered by gynaecological formulations (e.g. vaginal capsules, vaginal tablets, washings, etc.) or for intestinal use (e.g. powder divided in small envelopes, capsules, tablets etc.).

The formulations of the invention contain one or more lactic bacteria strains of the invention, in an effective quantity for the therapeutic or prophylactic intended treatment.

The claimed lactic bacteria strains, are useful in the local treatment of pathologies deriving from the abnormal proliferation of pathogens such as for instance diarrhea and/or bacterial vaginitis.

## Claims

1. Strains selected from:
*Lb*. *plantarum, 29.11.1996 LMG-P-17630*
*Lb. acidophilus, 15.01.1999 LMG-P-18806*
*Lb. delbreuckii, 15.01.1999 LMG-P-18805*
*Str. thermophilus, 15.01.1999 LMG-P-18807.*

2. Pharmaceutical compositions containing as active ingredient one or more strains of claim 1.

3. Compositions according to claim 2 in the form of gynecological or intestinal formulations.

4. Use of the strains according to claim 1 for the manufacture of a medicament for the treatment of diarrhea and/or bacterial vaginitis.

## Patentansprüche

1. Stämme, ausgewählt aus
Lb. plantarum, 29,11.1996 LMG-P-17630
Lb. acidophilus, 15.01.1999 LMG-P-18806
Lb. delbreuckii, 15.01.1999 LMG-P-18805
Str. thermophilus, 15.01.1999 LMG-P-18807.

2. Pharmazeutische Zusammensetzungen, umfassend als aktiven Bestandteil einen oder mehrere der Stämme gemäß Anspruch 1.

3. Zusammensetzung gemäß Anspruch 2 in Form gynäkologischer oder intestinaler Formulierungen.

4. Verwendung der Stämme gemäß Anspruch 1 zur Herstellung eines Medikamentes zur Behandlung von Diarrhöe und/oder bakterieller Vaginitis.

## Revendications

1. Souches choisies parmi:
*Lb. plantarium, 29.11.1996 LMG-P-17630*
*Lb. acidophilus, 15.01.1999 LMG-P-18806*
*Lb. delbreuckii, 15.01.1999 LMG-P-18805*
*Str. thermophilus, 15.01.1999 LMG-P-18807.*

2. Composition pharmaceutique comprenant en tant que principe actif une ou plusieurs souches selon la revendication 1.

3. Composition selon la revendication 2 sous la forme de formulations gynécologiques ou intestinales.

4. Utilisation des souches selon la revendication 1 pour la fabrication d'un médicament pour le traitement d'une diarrhée et/ou d'une vaginite bactérienne.
